# EUROPEAN PATENT SPECIFICATION

(11) **EP 3 122 284 B1**
(45) Date of publication and mention of the grant of the patent: **13.01.2021**
(21) Application number: 15768518.1
(22) Date of filing: 26.03.2015
(51) Int. Cl.: A61F 2/02, A61M 25/00

(54) **DEVICES FOR CLOSURE OF TRANSVASCULAR OR TRANSCAMERAL ACCESS PORTS**
VORRICHTUNGEN ZUM VERSCHLUSS VON TRANSVASKULÄREN ODER TRANSKAMERALEN ZUGANGSPORTS
DISPOSITIFS DE FERMETURE D'OUVERTURES D'ACCÈS TRANSVASCULAIRES OU TRANSCAMERALES

(30) Priority: 27.03.2014 US 201461971458 P; 22.11.2014 US 201462083192 P
(43) Date of publication of application: 01.02.2017
(73) Proprietor: Rafiee, Nasser, Andover, Massachusetts 01810 (US); Lederman, Robert J., Chevy Chase, Maryland 20815 (US); Rogers, Toby, Bethesda, Maryland 20814 (US); Ratnayaka, Kanishki, Washington, District of Columbia 20001 (US); Kocaturk, Ozgur, Rockville, Maryland 20852 (US)
(72) Inventor: Rafiee, Nasser, Andover, Massachusetts 01810 (US); Lederman, Robert J., Chevy Chase, Maryland 20815 (US); Rogers, Toby, Bethesda, Maryland 20814 (US); Ratnayaka, Kanishki, Washington, District of Columbia 20001 (US); Kocaturk, Ozgur, Rockville, Maryland 20852 (US)
(74) Representative: Guardian IP Consulting I/S
(86) International application number: PCT/US2015/022782
(87) International publication number: WO 2015/148821

(56) References cited:
- WO-A1-99/39646
- WO-A1-2013/159065
- US-A- 5 895 410
- US-A1- 2003 057 156
- US-A1- 2004 087 998
- US-A1- 2005 288 706
- US-A1- 2006 155 303
- US-A1- 2008 033 475
- US-A1- 2009 062 841
- US-A1- 2010 211 046
- US-A1- 2012 283 768
- US-A1- 2013 289 618

## Description

### BACKGROUND

### Field of the Disclosure

The present disclosure relates to a device and method for transcatheter correction of cardiovascular abnormalities, such as the delivery of prosthetic valves to the heart. The present disclosure further relates to implants for closing a caval-aortic iatrogenic fistula created by the introduction of a transcatheter device from the inferior vena cava into the abdominal aorta.

### Description of Related Art

Transcatheter procedures have been a milestone advance in modern medicine. Percutaneous or transthoracic catheters are advanced through the vascular system or other natural luminal orifices to effect mechanical remodeling through angioplasty or to effect occlusion or patency or valvular function through implants of self-expanding or balloon-expanding occluders, stents, and valves. These procedures can take the place of surgical repair in selected patients.

Percutaneous vascular occluders are limited because usually they require the operator to forego guidewire access between target chambers. Recent innovations permit vascular occluders to be engineered around a central guidewire lumen to enhance safety and versatility of the occluder procedure.

Recently, Halabi and colleagues (JACC 2013;61:1745), and thereafter Greenbaum and colleagues (Transcatheter therapeutics conference, San Francisco, 2013) reported a novel procedure to introduce large vascular devices into the aorta from the adjoining inferior vena cava. This enabled transcatheter aortic valve replacement in patients otherwise ineligible because of no surgical access to the thorax and insufficient iliofemoral artery caliber. The "caval-aortic" access port, as it is called, was closed using nitinol occluder devices marketed by St Jude Medical (Amplatzer muscular ventricular septal defect occluder or Amplatzer duct occluder) to close congenital cardiovascular defects. These devices are inadequately hemostatic, do not allow uninterrupted guidewire access, and are imperfectly suited for this application.

Transcatheter structural left heart procedures are generally performed through the femoral artery. However, femoral artery caliber or intravascular disease precludes or complicates vascular access in a significant minority of candidates for transcatheter aortic valve replacement or aortic endograft therapy. Moreover, the most frequent life-threatening complication of TAVR is vascular complications of large introducer sheaths placed in the femoral artery. Alternative transcatheter approaches to the heart would therefore be desirable. The present disclosure provides solutions for these and other problems as described herein.

US 2010/211046 discloses devices and methods for treating various target sites. For example, one exemplary device includes a tubular structure having proximal and distal ends and at least one layer of braided material configured to facilitate thrombosis. The tubular structure includes an end section at the proximal or distal end having a cross-sectional dimension that is larger than that of an opening of the target site. The device also includes a stiffener wire coupled to the tubular structure, wherein the wire is configured to extend at least partially through the opening and facilitate securing the end section over the opening. The tubular structure and stiffener wire include respective preset, expanded configurations and are configured to be constrained to respective reduced configurations for delivery to the target site and to at least partially return to their respective preset, expanded configurations at the target site when unconstrained.

US 2013/289618 discloses a septal occluder, such as one made from a polymer tube. The septal occlude can have portions on either side of a patent foramen ovale (PFO) or other septal defect. The portions on either side can be held in place with a catching mechanism that can take one of many forms. The tube can be made of bioresorbable materials. In one form the catching mechanism is a coil catching member, wherein when an operator releases the coil catching member, portions of the catching member that are not restrained by the inside diameter of center joints of the occluder expand thereby catching the occluder.

### SUMMARY OF THE DISCLOSURE

The purpose and advantages of the present disclosure will be set forth in and become apparent from the description that follows. Additional advantages of the disclosed embodiments will be realized and attained by the methods and systems particularly pointed out in the written description hereof, as well as from the appended drawings.

To achieve these and other advantages and in accordance with the purpose of the disclosure, as embodied herein, in one aspect, the disclosure includes embodiments that solve problems of caval aortic access based on considerable pre-clinical, animal, imaging, and clinical experience in caval-aortic access. These approaches differ substantially from the aforementioned prior art.

In certain embodiments, the problem of variable distance between aortic and caval access points is solved using a telescopic design as disclosed herein. The problem of inadequate hemostasis of aortic and caval access tracts, in some implementations, is solved using "billowing" nitinol weave to fill the vascular holes and by using multiple disks to occlude each vascular rent.

According to one aspect, a prosthesis is provided that comprises a radially expandable mesh body that is configured to self-expand into at least one disc after becoming radially unconstrained, the radially expandable mesh body defining a volume therein when expanded. The prosthesis further includes a resilient member in the form of a tension coil spring disposed within the volume of the mesh, the tension coil spring attached to a proximal end and distal end of the prosthesis along an axis that defines a central region of the prosthesis. The tension coil spring is configured to contract into a relaxed state when not being stretched that results in the prosthesis being shortened along the axis and expanded radially when the resilient member is relaxed.

The prosthesis can include a material disposed within the mesh that is configured to encourage coagulation when exposed to blood. For example, the material disposed within the mesh can include first and second fabric discs that are disposed within the at least one disc of the radially expandable mesh body to line the radially expandable mesh body with fabric. If desired, the prosthesis can further include a tubular fabric portion attached to at least one of the fabric discs. The tubular fabric portion can extend proximally into a neck region of the prosthesis.

In some implementations, the resilient member can be a coil spring that causes the prosthesis to collapse axially and the at least one disc to expand radially to prevent the prosthesis from being pulled axially through an opening it has been inserted through after it has been deployed. The resilient member is a tension coil spring. The coil spring can include at least two sections of different diameter. If desired, the coil spring can include at least three sections of different diameter. In some implementations, the coil spring can include at least three sections wherein each section has a diameter different than an adjacent section. If desired, the coil spring can include an enlarged central section for causing a neck portion of the prosthesis to bulge radially outwardly when the prosthesis is deployed.

If desired, the radially expandable mesh body can be configured to self-expand into at least two discs connected by the neck region after becoming radially unconstrained. A first disc of the two discs can be configured to mitigate high pressure leaks in an artery. A second disc of the two discs can be configured to mitigate low pressure leaks originating from a vein. The neck region can be configured to cooperate with the first and second discs to prevent leakage from the artery and the vein. At least one of the discs can include a plurality of radially oriented struts attached to the mesh of the at least one disc to enhance the axial compressibility of the prosthesis. The prosthesis can include one or more radially oriented struts attached to the discs at the distal and proximal faces of the prosthesis, that can extend from a radially central portion of the prosthesis to an outer periphery of the prosthesis.

The disclosure provides a system for delivering a prosthesis as defined in claim 11. The system can include one or more of an outer tubular sheath having a proximal end and a distal end and defining a first lumen therethrough along its length, an intermediate tubular member disposed at least partially within the first lumen, and having a proximal end and a distal end and defining a second lumen therethrough along its length. The system can include an inner elongate member disposed at least partially within the second lumen, and having a proximal end and a distal end. The system can further include a prosthesis removably mounted on the distal end of the intermediate tubular member. The prosthesis can include a radially expandable mesh body that is configured to self-expand into at least one disc after becoming radially unconstrained, the radially expandable mesh body defining a volume therein when expanded. The prosthesis can further include a resilient member disposed within the mesh that is attached to a proximal end and distal end of the prosthesis along an axis that defines a central region of the prosthesis. The resilient member can be configured to cause the prosthesis to shorten along the axis and expand radially when the resilient member is relaxed. Distal movement of the inner tubular member against a portion of the prosthesis can cause it to collapse radially and elongate axially so as to permit the prosthesis to be collapsed after deployment, adjusted and redeployed or retracted into the outer tubular member and removed.

If desired, the inner elongate member can be a tubular member, such as a hypotube or polymeric tubular member or composite tubular member configured to permit a guidewire to pass therethrough. The resilient member can be a coil spring, that causes the prosthesis to collapse axially and the at least one disc to expand radially to prevent the prosthesis from being pulled axially through an anatomical opening it has been delivered through after it has been deployed.

The resilient member is a tension coil spring In some implementations, a distal end of the outer tubular member can be cut at an angle that is oblique with respect to a central axis defined by the system. The system can include a steering mechanism to articulate the distal end of the outer tubular member, such that the oblique cut of the distal end of the outer tubular member can facilitate navigation of the distal end of the system, such as through a caval-aortic iatrogenic fistula created by the introduction of a transcatheter device from the inferior vena cava into the abdominal aorta.

If desired, the coil spring can include an enlarged central section for causing the neck portion of the prosthesis to bulge radially outwardly when the prosthesis is deployed. In some implementations, the radially expandable mesh body can be configured to self-expand into at least two discs connected by the neck region after becoming radially unconstrained. A first disc of the two discs can be configured to mitigate high pressure leaks in an artery. A second disc of the two discs can be configured to mitigate low pressure leaks originating from a vein. If desired, the neck region can be configured to cooperate with the first and second discs to prevent leakage from the artery and the vein. In some implementations, the prosthesis can include a material disposed within the mesh that is configured to encourage coagulation when exposed to blood.

The disclosure further provides various prostheses including a radially expandable mesh body that is configured to self-expand into at least one disc after becoming radially unconstrained, the radially expandable mesh body defining a volume therein when expanded. The prosthesis further includes at least one tether attached to the radially expandable mesh body that is configured to cause the radially expandable mesh body to collapse radially when tension is applied to the at least one tether.

If desired, the radially expandable mesh body can be configured to self-expand into at least two discs connected by a neck region after becoming radially unconstrained. A first disc of the two discs can be configured to mitigate high pressure leaks in an artery. A second disc of the two discs can be configured to mitigate low pressure leaks originating from a vein. The neck region can be configured to cooperate with the first and second discs to prevent leakage from the artery and the vein.

In some implementations, the radially expandable mesh body can be configured to self-expand into at least three discs connected by two neck regions after becoming radially unconstrained. A first disc of the two discs can be configured to mitigate high pressure leaks in an artery. A second disc of the two discs can be configured to mitigate low pressure leaks originating from a vein. The neck region can be configured to cooperate with the first and second discs to prevent leakage from the artery and the vein.

If desired, the radially expandable mesh body can be configured to self-expand into at least four discs connected by three neck regions after becoming radially unconstrained. A first disc of the two discs can be configured to mitigate high pressure leaks in an artery. A second disc of the two discs can be configured to mitigate low pressure leaks originating from a vein. The neck region can be configured to cooperate with the first and second discs to prevent leakage from the artery and the vein. If desired, the at least one tether can be threaded through all of the discs to cause all of the discs to collapse radially inwardly upon applying sufficient tension to the at least one tether. In some implementations, the prosthesis can include a material disposed within the mesh that is configured to encourage coagulation when exposed to blood.

In further accordance with the disclosure, any prosthesis disclosed herein can be formed at least in part from a composite wire. In some embodiments, the composite wire can be drawn filled wire. For example, the drawn filled wire can include a first material, and a second material in a different region of the drawn filled wire that has greater radiopacity than the first material. The first and second materials can include metallic components and/or bioresorbable components. If desired, the second material can be located along a core region of the wire, and first material can surround or substantially surround the first material. The first material can include a NiTi alloy, and the second material can include platinum, for example.

In further implementations, the disclosure provides an implantable prosthesis that includes an inflatable bioresorbable body having a proximal end and a distal end, the body being configured to be expanded radially by directing fluid into the body. The prosthesis further can include at least one radially expandable strut attached to each of the proximal end and distal end of the body. Each of the struts can be configured to expand outwardly to prevent the prosthesis from being pulled through an anatomical opening into which it has been inserted.

If desired, the aforementioned prosthesis can further include a coupling located at the proximal end of the prosthesis configured to be attached to a delivery system. The coupling can be configured to permit inflation fluid to pass therethrough.

In a further embodiment, a prosthesis is provided as described herein having a mesh body that is configured to self-expand into at least two discs connected by a neck region after becoming radially unconstrained. A first disc of the at least two discs can be configured to mitigate leaks, and a second disc of the at least two discs can be configured to cause appropriate positioning of the prosthesis in the presence of cardiovascular motion. Such a prosthesis can be used, for example, to address high pressure leaks from an artery or a cardiac chamber. In some implementations, such a prosthesis can be used to address a ventricular septal defect (VSD) (i.e., a hole in the heart). This is a common heart defect that's present at birth (congenital). The hole occurs in the wall that separates the heart's lower chambers (septum) and allows blood to pass from the left to the right side of the heart. The oxygen-rich blood then gets pumped back to the lungs instead of out to the body, causing the heart to work harder. The prosthesis can be delivered and deployed into the defect and deployed, sealing the hole.

In further embodiments, such a prosthesis can be used for various transcardiac applications, wherein the second disk assures retention in position of the prosthesis. For example, such a prosthesis can be used to seal an access opening through the aortic arch that is formed for accessing the aortic valve after the valve is replaced. Similarly, such an approach can be used to seal openings formed in lumenal or vascular walls such as apical access procedures for sealing openings formed through the ventricular wall, for sealing openings formed in a septum (e.g., patent foramen ovale (PFO)) and the like.

It is to be understood that both the foregoing general description and the following detailed description are exemplary and are intended to provide further explanation of the embodiments disclosed herein.

The accompanying drawings, which are incorporated in and constitute part of this specification, are included to illustrate and provide a further understanding of the method and system of the disclosure. Together with the description, the drawings serve to explain the principles of the disclosed embodiments.

### BRIEF DESCRIPTION OF THE DRAWINGS

The foregoing and other objects, aspects, features, and advantages of exemplary embodiments will become more apparent and may be better understood by referring to the following description taken in conjunction with the accompanying drawings, in which:
Figs. 1A-1D depict a distal portion of an illustrative delivery system for delivery of a prosthesis for closure of transvascular or transcameral access ports and the prosthesis itself.
Figs. 2A-2D illustrate various aspects of the prosthesis delivered by the device of Fig. 1.
Fig. 3A illustrates an embodiment of a further prosthesis in accordance with the disclosure.
Figs. 3B-3C illustrate examples of prior art prostheses.
Figs. 4A-4C illustrate a further embodiment of an implantable device in accordance with the disclosure mounted on the distal end of a delivery system.
Fig. 4D illustrates a further embodiment of an implantable device in accordance with the disclosure.
Figs. 5A-5C illustrate a prosthesis mounted on the distal end of a delivery system, showing articulation of the delivery cable shaft.
Figs. 6A-6C illustrate variations of windings that can be used to help form the prosthesis.
Fig. 7-8 illustrates a portion of a delivery system in accordance with the disclosure without a prosthesis mounted thereon and with an outer portion of the system removed.
Fig. 8 is an illustration of an example of a delivery system with a prosthesis mounted thereon.
Figs. 9A and 9B illustrate a further adjustable, compliant, maneuverable, retrievable and repositionable four disc/lobe closure system.
Figs. 10A-10B illustrate a three disc/lobe embodiment wherein a central disc is located between the aorta and inferior vena cava.
Fig. 11A-11B illustrate a four disc and three disc embodiment of a prosthesis.
Fig. 12A-12B illustrate the prosthesis in a deployed condition with the end disc flattened.
Fig. 13A-13B illustrate the prosthesis in a deployed condition with the end disc flattened.
Fig. 14A-14B illustrate the prosthesis and delivery catheter with tethers running through all four and three prosthesis and into a guiding sheath of the delivery catheter.
Figs. 15A-15B and Figs. 16A-16B illustrate a complete deployment of a three disc embodiment from beginning to end.

### DETAILED DESCRIPTION

Reference will now be made in detail to the present preferred embodiments of the disclosure, examples of which are illustrated in the accompanying drawings. The method and corresponding steps of the disclosed embodiments will be described in conjunction with the detailed description of the system.

The exemplary embodiments illustrated herein can be used to more effectively close transvascular or transcameral access ports.

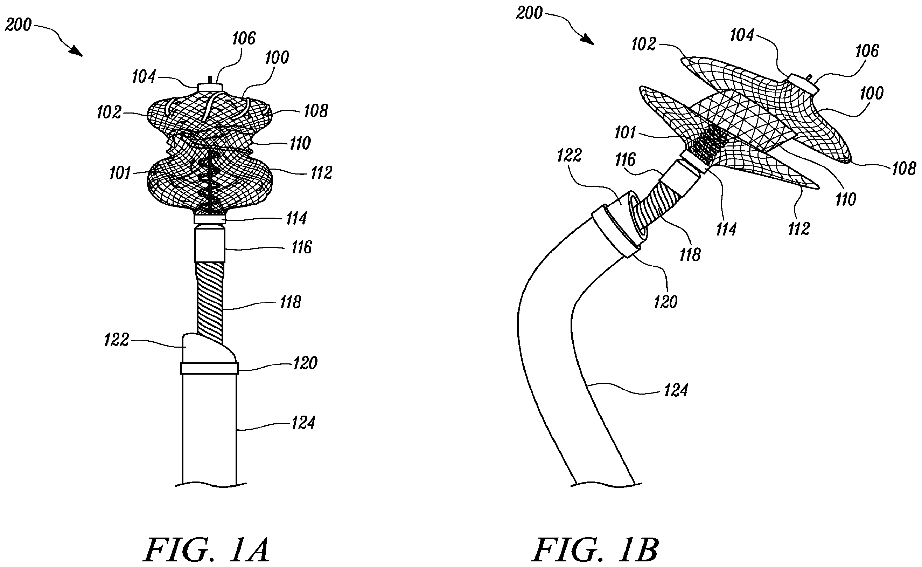

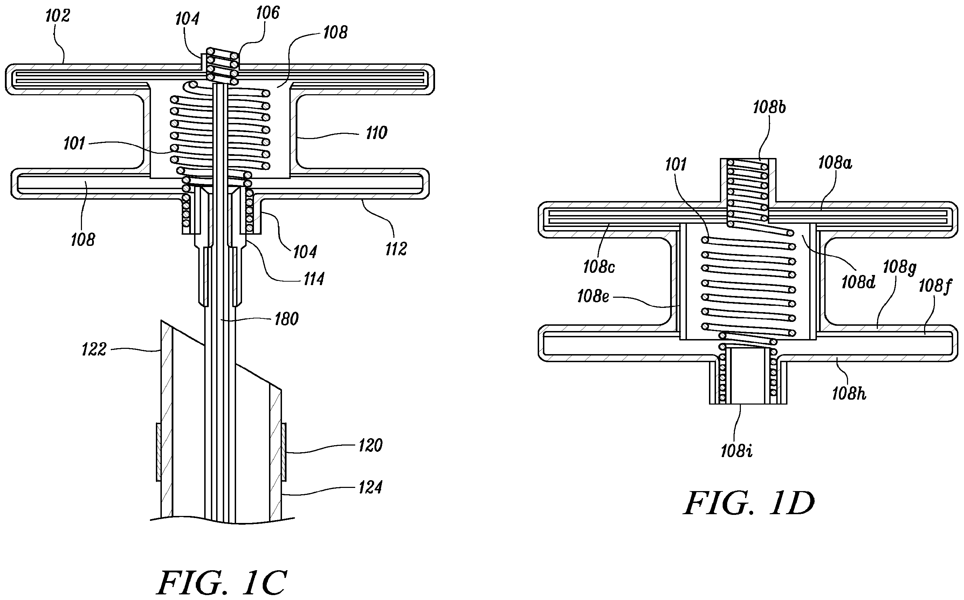

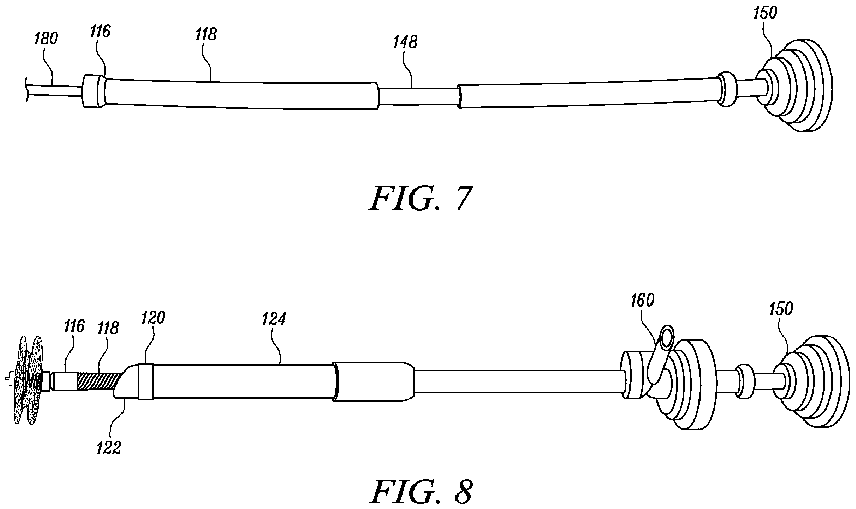

For purposes of illustration, and not limitation, as embodied herein and as illustrated in Fig. 1, a delivery system 200 is provided including prosthesis 100 mounted thereon. An illustration of a full exemplary system can be seen in each of Fig. 7 and Fig. 8, which are discussed in further detail below.

As illustrated, the distal region of the system 200 includes a distal end of an outer tubular member 124 that can be introduced through a lumen of a guiding catheter (not shown) that is used to deliver a prosthesis or perform some other function via a transvascular or transcameral access port. The distal end of the outer tubular member 124 is preferably provided with a distal radiopaque marker 120, such as one made at least in part from silver, gold, platinum or other radiopaque material, as desired. The distal tip 122 of the outer tubular member can be cut on a bevel to facilitate guiding the device across the wall of the inferior vena cava into the aorta, for example. The bevel may be at any suitable angle, but is preferably offset from a central axis of the catheter by an angle between about 30 degrees and about sixty degrees, or any angular increment therebetween of about one degree. In an illustrative embodiment, the angle can be about 45 degrees. The outer tubular member can be articulable or steerable at its distal end to facilitate maneuverability of the system.

Outer tubular member 124 may be made from a variety of materials. For example, the sheath 120 can include a multi-layered co-extrusion, such as those described in U.S. Pat. No. 6,464,683 to Samuelson or U.S. Pat. No. 5,538,510 to Fontirroche.

Any surface of various components of the catheters described herein or portions thereof can be provided with one or more suitable lubricious coatings to facilitate procedures by reduction of frictional forces. Such coatings can include, for example, hydrophobic materials such as Polytetrafluoroethylene ("PTFE") or silicone oil, or hydrophilic coatings such as Polyvinyl Pyrrolidone ("PVP"). Other coatings are also possible, including, echogenic materials, radiopaque materials and hydrogels, for example.

Within the outer tubular member 124 of the delivery system 200 a tubular delivery cable, or intermediate tubular member 118, is slidably disposed defining therethrough a central lumen along its length for slidably receiving a pushrod 180 therethrough, discussed in detail below. A distal region of the intermediate tubular member 118 can be configured to be of a lower stiffness, or durometer, than a proximal region of the cable to make it easier to articulate the distal end of the system, such as embodiments wherein the outer tubular member 124 have an articulable distal end or region. As illustrated, the intermediate tubular member 118 terminates in a coupling 116 for attachment to the prosthesis 100. The illustrated coupling 116 is a female member that receives a corresponding male coupling portion 114 on the prosthesis 100, but it will be appreciated that the coupling 116 on the delivery system can be male and that the coupling on the prosthesis can be female. In some implementations, a female coupling can be provided on the prosthesis that is defined by the inside of a coiled member, discussed in further detail below, that is received by a male threaded coupling on the delivery system. The coupling may be a threaded coupling but can also be a twist and lock coupling or the like.

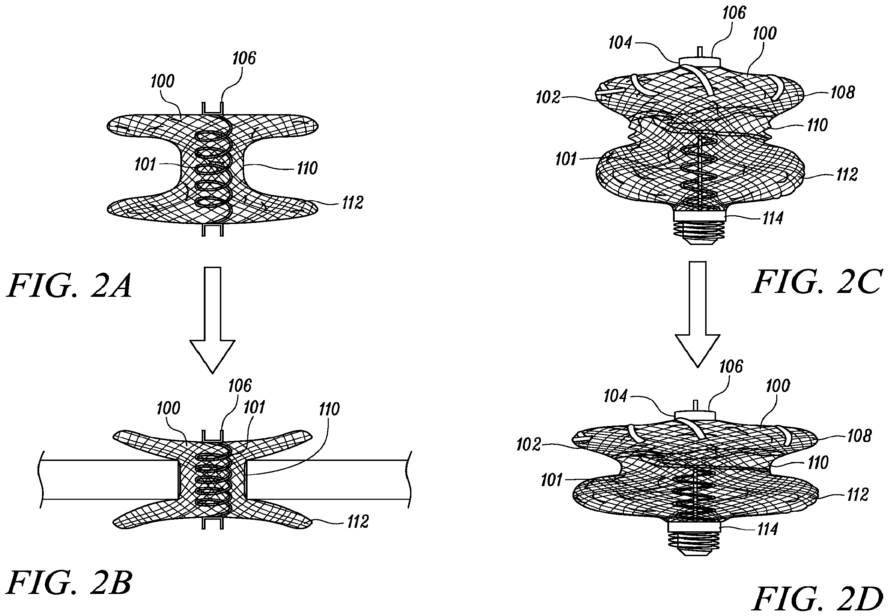

As further illustrated in Figs. 1A-1B, a first exemplary embodiment of a prosthesis 100 is provided having a proximal end that connects to the delivery system, and a distal end 104 through which a guidewire can extend via a guidewire lumen 106. As illustrated, the prosthesis 100 includes an interior coil tension spring 101 that is configured to be contracted into a relaxed state when not being stretched. This results in the prosthesis being in a shortened, compressed state when relaxed. This is most evident in Fig. 1C, showing a compressed prosthesis 100 when the tension spring 101 is in a relaxed, unelongated condition. By stretching the coil spring, such as by pulling both ends of the prosthesis apart, the prosthesis takes on a more elongate profile with a smaller radial profile, such as depicted in Figs. 1A or 2C.

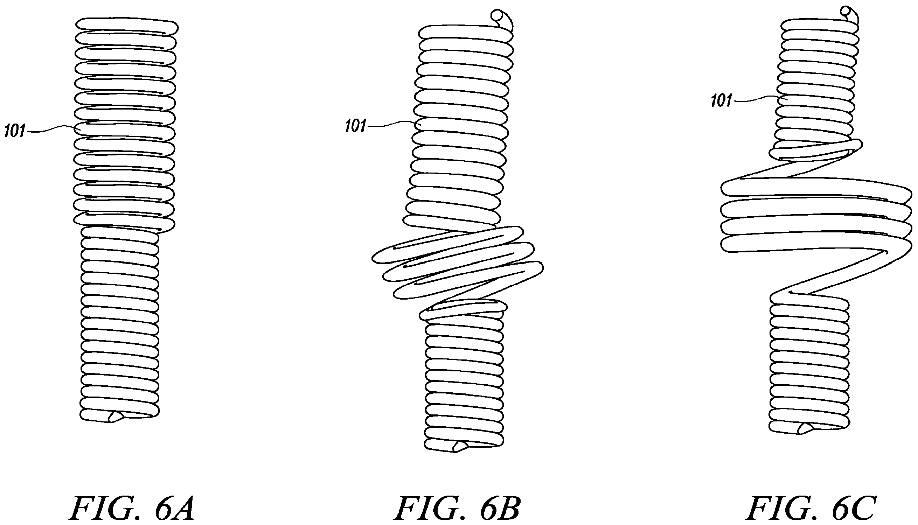

The coil spring 101 can have a substantially uniform outer and inner diameter along its length from a proximal end of the prosthesis to the distal end of the prosthesis. Alternatively, as illustrated in Figs. 6A, the coil spring 101 can have a first portion that has a larger diameter than a second portion, wherein the smaller diameter portion can be directed toward the proximal or distal end of the prosthesis. As illustrated in Fig. 6B, the coil spring can have three regions of different diameters, wherein a central region of the coil spring can have an enlarged diameter with respect to the diameter of either end portion. The end portions can be of the same or different diameters. Having an enlarged central portion can help prevent leakage after the prosthesis is installed as the coil can urge against the graft and/or mesh material within the adjustable neck 110 region of the deployed prosthesis between the lumens to cause the neck to bulge outwardly against the outer lumen walls to facilitate sealing and to help prevent leakage. The windings of the coil spring that is attached to the high pressure disc on the distal end of the prosthesis can be sized and shaped to prevent the hypotube from passing out of the distal end of the prosthesis, but to permit the guidewire to pass through. Similarly, the portion of the coil spring at the proximal end of the prosthesis that faces the delivery system can have a coil spring that is configured to be threaded onto a male end of the intermediate tubular member 118 to attach the prosthesis to the delivery system.

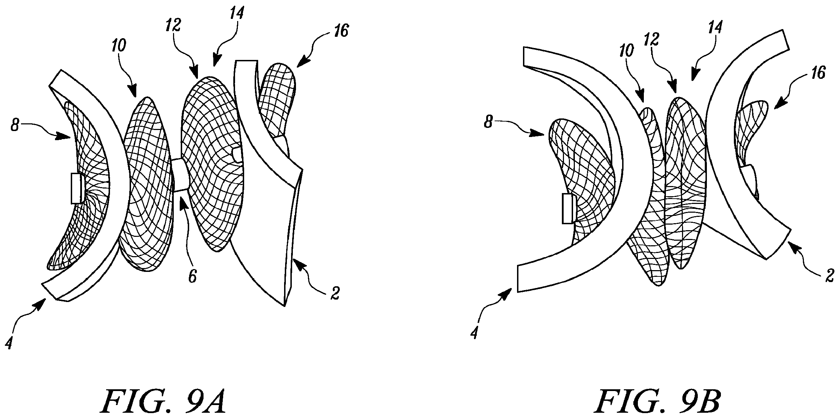

It will be appreciated that while a coil spring is primarily illustrated herein and is preferred, other resilient or elastic members can be used in place of the coil spring, or the prosthesis may instead by provided with retractable tethers (discussed in detail below with respect to Figs. 9 onward). For example, a single or a loop Elastic band that is attached to the radially expandable discs can be used, and/or any suitable material that can act as a tension spring and have a suitably low profile.

The prosthesis 100 further includes a mesh covering that may be braided from a variety of materials such as NiTi alloys or bioresorbable materials. It should be noted that, in some implementations, the prosthesis can be made from bioresorbable materials in its entirety. Suitable bioresorbable materials and techniques for construction can be found, for example, in U.S. Patent Application Serial No. 11/398,363, filed April 4, 2006, and U.S. Patent Application Serial No. 14/461,159, filed August 5, 2014.

The mesh covering preferably defines at lease one proximal lobe, or disc 112 and at least one distal lobe, or disc 102 joined by a narrowed neck 110 region that can be adjustable in radial dimension so as to permit a custom fit during implantation to minimize or eliminate leakage from the aorta and IVC. The mesh covering is joined at each of the proximal and distal ends to the respective proximal and distal ends of the coil spring. If desired, the disc 102 can be a high pressure endolumenal disc configured for placement against an inner arterial wall and disc 112 can be a low pressure disc configured for placement, for example, against an inner wall of the inferior vana cava.

The interior of the mesh can be filled with a woven graft material 108 and/or an elastomer with a coagulating coating, such as polyethylene glycol (PEG), or other non-thrombogenic, bio-inert polymer or polymer precursor.

For example, as illustrated in Fig. 1D, which presents a cross section of prosthesis 100, the distal face of the distal lobe or disc can include a first disc shape graft portion 108a that has a continuous surface except for a small hole or aperture 108b at the center thereof for surrounding the distal end of the coil spring 101 where it meets the mesh to permit the guidewire to pass through the distal end of the prosthesis. This first disc shaped portion 108a can be joined about its outer periphery (e.g., by weaving or stitching) to a second disc shaped portion 108c which also defines therein a central aperture 108d which may be slightly larger than 108b to permit passage therethrough of the coil spring which in turn is sized and shaped to permit passage therethrough of a pushrod (e.g., a stainless steel or NiTi hypotube, or polymeric (e.g., PEEK) or composite (e.g., carbon fiber) tubular member) of the delivery system containing the guidewire (discussed below). A further tubular graft portion 108e can be attached to and depend in a proximal direction from the proximal face of second disc shaped portion 108c to line the neck region of the prosthesis 100 and to surround the central region of the coil spring 101. If desired, portion 108e can be stitched at one or more locations to the mesh structure. In some implementations, the graft material 108 can still further include a third disc-shaped portion 108f attached to the proximal end of tubular portion 108e also defining a central aperture therein 108g for permitting passage of the coil 101. Disc 108f can similarly be joined about its periphery via weaving or stitching to a fourth, proximal disc 108h defining therein a central aperture 108i, which in turn surrounds the proximal end of the spring where it meets the proximal portion of the mesh to seal around the spring. The outer periphery of the four aforementioned discs may be stitched to each other and to the mesh to ensure proper registration of the mesh with the graft material.

As further illustrated in Fig. 1C, a pushrod 180 is slidably disposed within the lumen of the intermediate tubular member 118. The pushrod includes a proximal end attached to an articulable proximal handle (see e.g., Figs. 7 and 8) and a distal end that abuts an inner surface of the distal end portion of the prosthesis 100. Specifically, the distal central opening of prosthesis 100 is large enough to permit a guidewire to pass therethrough, such as between 0.010 and 0.060 inches or other suitable diameter. Preferably, the aperture is small enough for the distal end of the pushrod 180, which defines a central lumen to slidably house the guidewire, to not pass through the opening, and instead urge against the inner distal surface of the prosthesis. Instead, the distal end of the pushrod (or push tube, as desired), abuts an inner distal surface of the prosthesis at or near the location of the opening. For example, the inner distal surface of the prosthesis can define a shoulder about the opening that the push rod pushes against, or if desired, the windings of spring 101 can be such that the most distal windings permit the guidewire to pass through, but not the push rod/push tube.

Fig. 2A is a schematic cross sectional representation of the prosthesis 100 in an extended or longitudinally stretched state wherein the coil spring 101 is in a stretched condition, and Fig. 2B illustrates the prosthesis in a relaxed condition in situ after installation gripping both sides of a lumenal passage. Fig. 2C similarly depicts an outer view of a prototype prosthesis 100 in an expanded condition wherein the push rod/push tube is urged distally within the delivery system (not shown) against the distal end of the prosthesis to cause the coil spring to stretch longitudinally. Fig. 2D illustrates the same prosthesis in a relaxed condition after the push rod/push tube is withdrawn. As can be appreciated, the coil spring 101 forces each of the lobes or discs including the exterior mesh with graft material inside to flatten and better appose the wall of the vessel (or chamber, depending on the application) and thereby better achieve hemostasis.

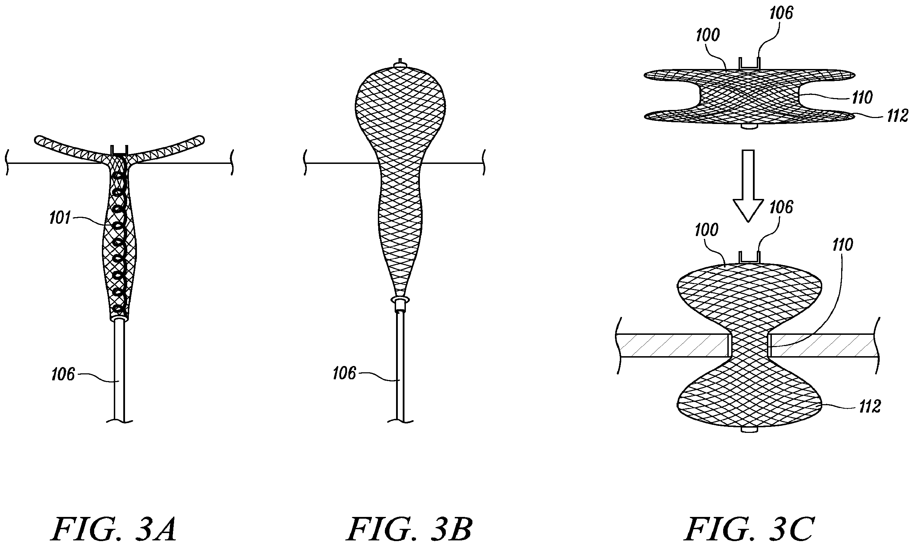

Fig. 3 illustrates relative performance between a disclosed embodiment and prior art devices. For example, an exemplary embodiment having a first disc section constructed as described above is presented in Fig. 3a. This embodiment resists pull-through the lumenal or chamber wall because the spring, or elastic member, is attached to the center of the distal or "high-pressure" disk (that could be located within an artery, for example), which causes the distal disk to flatten. Prior art devices (Figs. 3B & 3C), such as Amplatzer Duct Occluder product, does not have such an elastic member or spring, and therefore assumes an oblong configuration during the retraction phase of deployment (arrow) and therefore is susceptible to inadvertent pull-through, which naturally leads to a potentially dangerous situation for the patient.

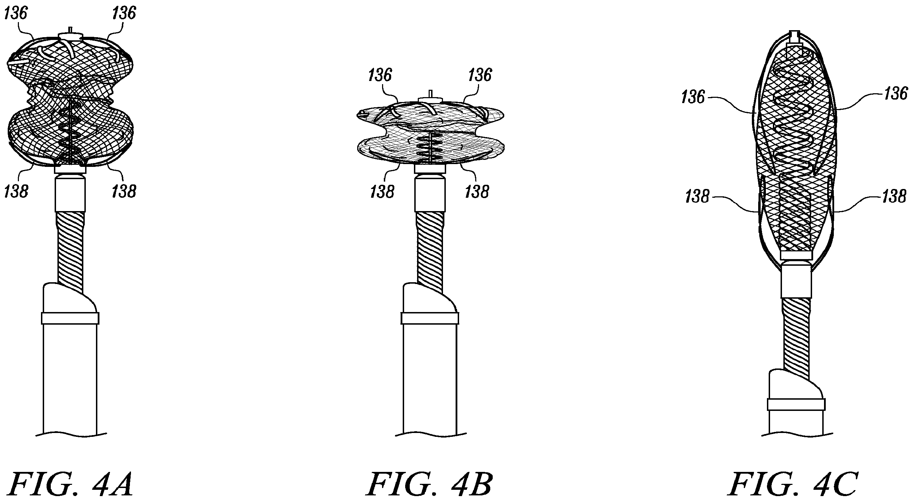

Figs. 4A-4C illustrate a further embodiment of a prosthesis that, in addition to having all of the aforementioned structural features, additionally include additional elongate radially oriented struts or "wings" 136, 138 that are attached to the mesh of the prosthesis at the distal and proximal faces of the prosthesis, extending from a radially central portion of the prosthesis to an outer periphery of the prosthesis. These struts enhance the collapsibility of the prosthesis under the action of the spring or elastic member. While these wings or struts can be constructed as loops, they can be made in any desirable or suitable manner. Fig. 4A illustrates such a prosthesis in a longitudinally expanded configuration wherein the mesh envelope is stretched longitudinally over the graft material and spring, whereas Fig. 4B illustrates the prosthesis in a relaxed condition wherein it can seal against one or more lumenal walls. Fig. 4C further illustrates the prosthesis in a fully longitudinally expanded configuration wherein the push rod/push tube is fully extended causing the prosthesis to collapse radially inwardly so that it can be inserted into a delivery sheath. Significantly, this design permits the device to be retrieved and removed, or removed and repositioned and reimplanted and moved as desired. In other words, the combination of the elastic member or spring and delivery system with a push rod or push tube greatly enhances deliverability and placement of the prosthesis.

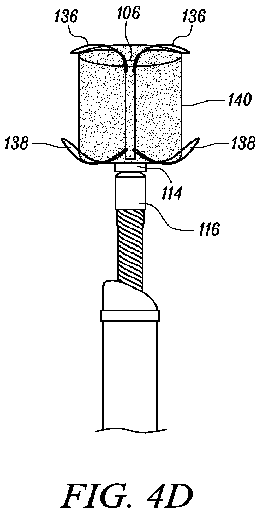

Fig. 4D illustrates an alternative embodiment of a prosthesis 140 that includes a main body formed from an inflatable, preferably bioresorbable material that is configured to seal a transcameral or transvascular access port, or other anatomical opening to be sealed. As illustrated, the prosthesis 140 has a proximal end attached to coupling 114 that in turn is removably attached to a coupling 116 located at the distal end of the intermediate tubular member 118. A lumen (not visible) passing through member 118 can carry fluid therethrough for inflating prosthesis 140 during delivery. Fluids, such as biodegradable polymer, resin or saline can be used to inflate prosthesis 140.

Fluid ports (not visible) can be provided in each of members 114, 116 to facilitate the inflation. Optionally, a guidewire port 106 can additionally be included. Wings 136, 138 can also be included to hold against the interior surfaces of the aorta and inferior vena cava, for example, while the inflatable body of the prosthesis 140 spans the gap between the two vessels and protrudes slightly into each vessel. The prosthesis 140 can be radially compressed within the distal end of outer tubular member 124 as with prosthesis 100. The compressed prosthesis 140 can be delivered to the site at which it is to be implanted, and the wings 136 can be deployed inside the aorta, for example, or other first location. The outer tubular member/sheath 124 can be retracted proximally thereby exposing the entire prosthesis 140 to the surrounding anatomy. A fluid actuator (e.g., fluid plunger that is actuated linearly or rotationally with a rotating handle driving screw) can then be depressed/actuated causing inflation fluid to be directed through the delivery system and into the prosthesis 140. The prosthesis 140 can be inflated to a desired extent to block leakage, and the wings 138 can be deployed (before, during or after inflation), causing the prosthesis to be lodged within the desired location. The wings/struts 136/138 could be wire loops that pass through the body of prosthesis, or can be mounted on either end of the prosthesis 140. Wings 136/138 are preferably shaped so they can be easily collapsed and retrieved into the delivery system.

If it is desired to move or remove the prosthesis 140, the fluid can be evacuated from the prosthesis by moving the fluid actuator in the opposing direction. The prosthesis can then be repositioned and implanted, or withdrawn into the distal end of outer tubular member 124, as desired. If desired, a push rod or push tube can be used to assist in retrievability of the prosthesis 100.

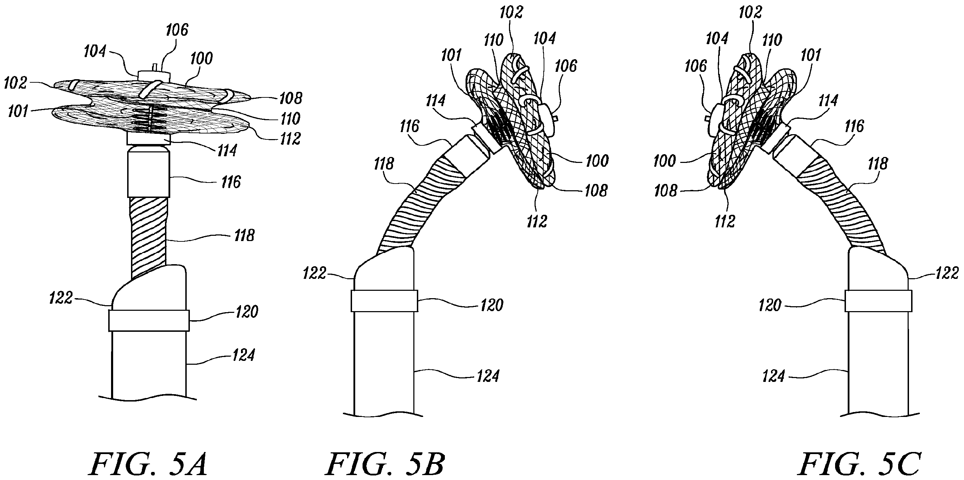

Figs 5A-5C further illustrate an embodiment of the prosthesis in a collapsed state on a delivery system in various articulated/steered positions allowing for adapting to the oblique angle of the device necessary for deployment and final release of the device.

Figure 7 is an end to end illustration of a portion of an example of delivery system in accordance with the disclosure without the prosthesis mounted thereon and with the outer tubular member removed. As can be seen in Figs. 7 and 8, the device itself, as well as the outer tubular member, intermediate tubular member, and push rod or push tube each have proximal end attached to a handle or control knob and a distal end. The intermediate tubular member as illustrated in Fig. 7 includes a relatively stiff proximal portion attached to a back end 150, and a transition segment 148 that is in turn attached to a distal flexible segment that terminates in coupling 116.

As illustrated in Fig. 8, the outer tubular member 124, or main delivery catheter, includes a back end 160 including a handle and steering knob configured to articulate the distal end of main delivery catheter/outer tubular member 124 that is attached to a proximal tubular region which in turn is connected to a distal tubular region terminating in beveled tip 122. The actuator 160 can take on a variety of forms, such as those depicted in U.S. Pat. No. 6,488,694 to Lau and U.S. Pat. No. 5,906,619 to Olson.

Before the system is introduced into the patient via a guiding catheter (not shown), the push rod 180 is fully distally extended to radially collapse the prosthesis, after which the intermediate tubular member can be withdrawn into the distal end of the main delivery catheter 124. The intermediate tubular member 118, or delivery cable shaft, thus preferably has variable stiffness along its length with a softer distal segment allowing for adapting to the oblique angle of the device necessary for deployment and final release of the device.

However, the present disclosure provides additional embodiments. For example, if desired, the prosthesis can be provided with more than two discs or lobes.

For purposes of illustration, and not limitation, Figs. 9A and 9B illustrate a further adjustable, compliant, maneuverable, retrievable and repositionable four disc/lobe closure system that resembles the two lobe system discussed above, except that two additional discs or lobes are provided between the proximal and distal lobes. While the discs are depicted as being formed from a NiTi alloy, it will be appreciated that any suitable material can be used.

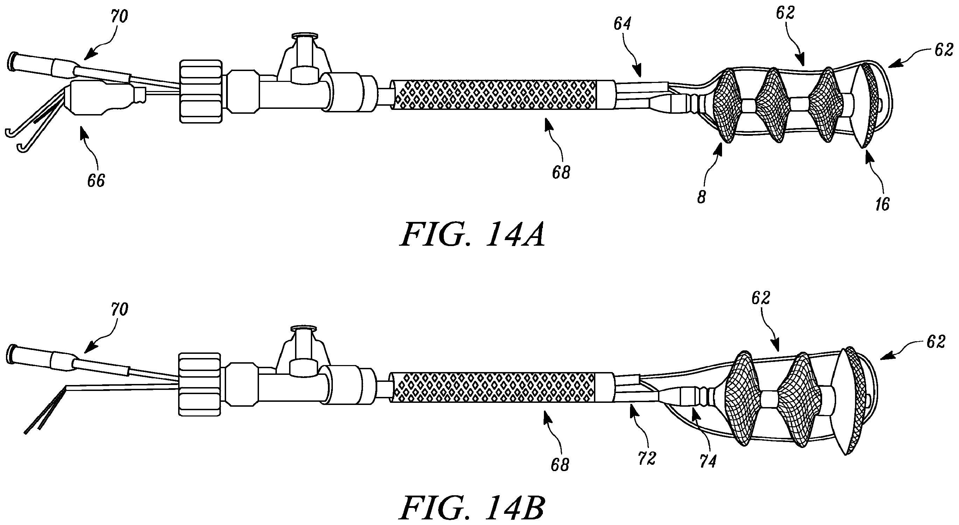

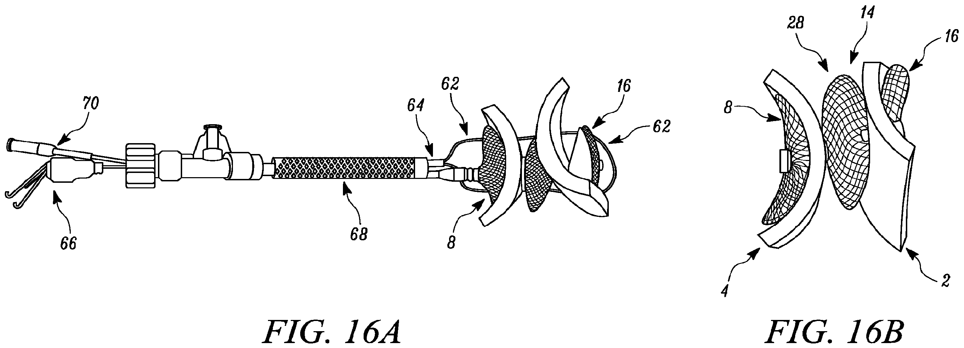

As illustrated in Figs. 9A and 9B, in an installed formation, the prosthesis includes a first high pressure or artery facing disc 16 that is deployed in the aorta, for example. The caval wall 4 and arterial wall 2 are presented with the prosthesis mounted therein. A next proximal disc 12 is provided for deployment against the outer wall of the aorta. A marker band 6 is also provided to enhance retention of the prosthesis. A third external caval disc 10 is provided for urging against the exterior of the inferior vena cava, and a fourth disc 8 is provided to seat within the IVC. One or more of the four discs can be provided with an exterior curvature or taper 14 that facilitates sealing, and all four discs can be formed from a mesh as with embodiment 100. A spring need not be located within the prosthesis of Fig. 9, but it can be. Collapsing of the prosthesis can be facilitated with tethers 62 that run through all four and three prosthesis and into a guiding sheath 64 of the delivery catheter as illustrated in Fig. 14A-B. As illustrated in Figs. 16A-B, the tethers can be withdrawn and pulled and the delivery cathether can be held fast to tighten the tethers until all leaks are stopped. The tethers can then be tied off or clipped, and the delivery system can be removed accordingly.

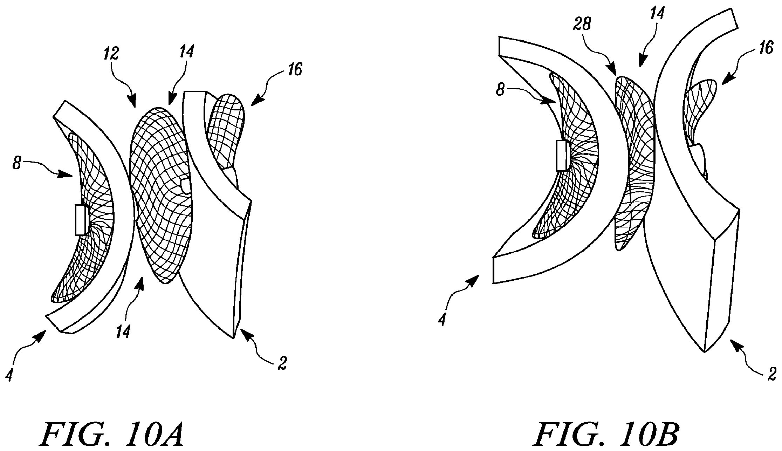

Fig. 9A shows the prosthesis after deployment but before the tethers are cinched, while Fig. 9B shows the tethers after cinching. Figs. 10A-B similarly show a three disc/lobe embodiment wherein a central disc 28 is located between the aorta and inferior vena cava. The construction is otherwise the same as the embodiment of Fig. 9, and may be constructed with an interior spring if desired as with embodiment 100 if desired, and/or can be provided with tethers as the embodiment of Figs. 9A-9B. The embodiment of Figs. 10A-B may also be provided with a guidewire lumen 30.

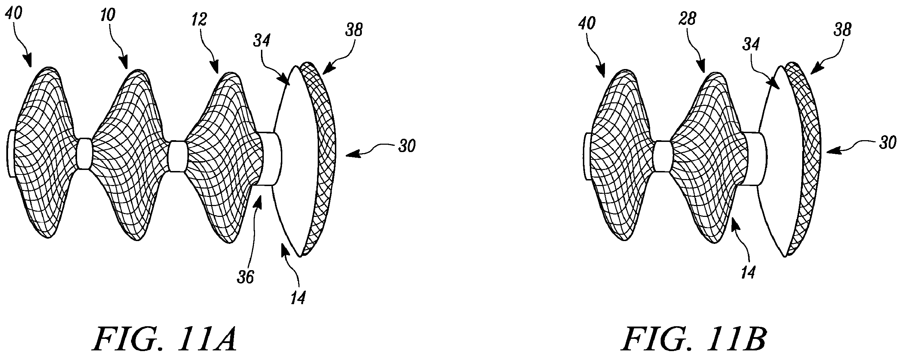

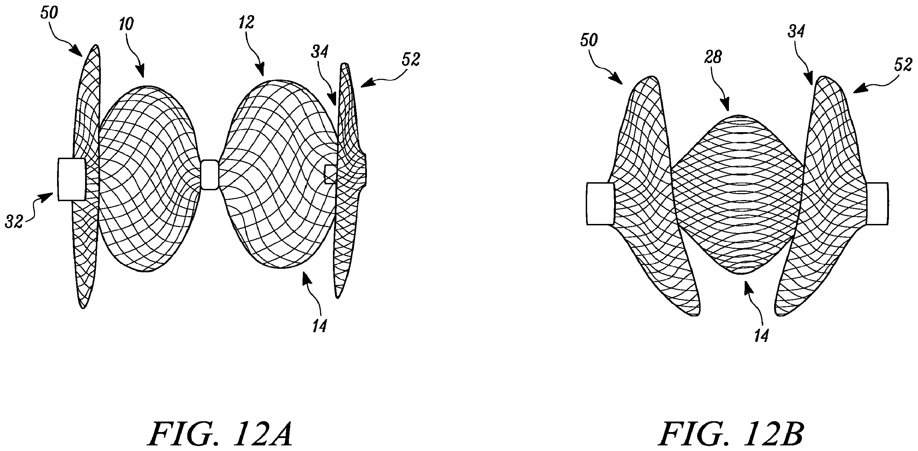

Fig. 11A depicts a four disc embodiment of a prosthesis wherein a radiopaque and/or elastomer covered neck 36 is provided between the high pressure disc 38 and the second disc 12, wherein the elastomer 34 is designed to help prevent leakage. Also visible are the low pressure caval disc 40 and third disc 10, wherein discs 10, 12 and 40 each have radiopaque markers disposed therebetween. Fig. 12A shows the prosthesis in a deployed position wherein the end discs are flattened and the middle discs are not fully flattened. Fig. 11B similarly provides a three disc version wherein like reference numbers indicate like structures.

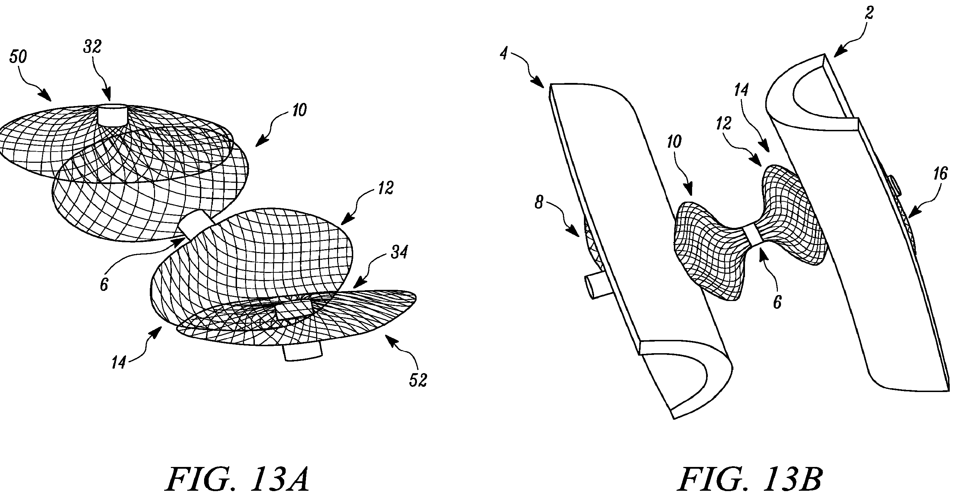

Figs. 12A illustrates a four disc embodiment in a deployed condition wherein the caval disc 50 and aortic disc 52 are compressed and fixed, and the intermediate discs 10, 12 are expanded to express a taper 14 to facilitate sealing and prevent leakage. Fig. 12B illustrates a three lobed prosthesis wherein the caval 52 and aortic 50 discs are fully deployed and flattened, and further wherein the central disc 28 is deployed to define a tapered sealing surface 14. Figs. 13A-B show a further variant of a four disc embodiment in a semi deployed condition and in a simulated installed condition in anatomy.

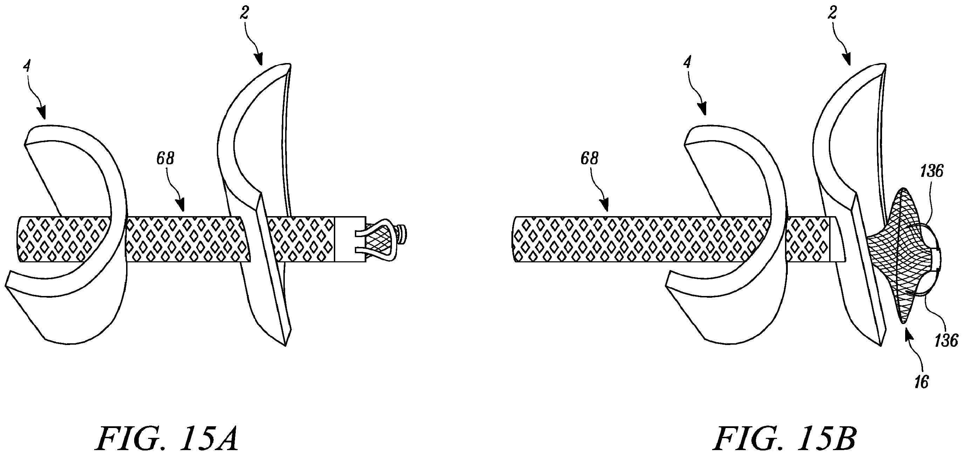

Figs. 14A-14B illustrate four and three disc prosthesis embodiments respectively with tethers 62 routed through them, wherein the aortic disc 16 is located at a distal end of the assembly, and a most proximate disc 8 is also provided. The tethers 62 are directed through the prosthesis, and then proximally through a tubular member, or tether lumen 64, toward the proximal end of the delivery system 68 through a port and/or handle 66. A handle 70 is further provided that is attached to an elongated member or closure holding shaft 72 (e.g., tube or rod) that is attached to the prosthesis via a removable coupling, such as a holding, releasing and/or retrieval articulating screw with wire lumen. Figs. 15-16 show a complete deployment of a three disc embodiment from beginning to end.

In further accordance with the disclosure, embodiments are also provided, but not specifically illustrated, that adds the tethering features of the embodiments of Figs. 14-16 to any other embodiment disclosed herein, including but not limited to the embodiments of any of Figs. 1-13, whether or not such embodiments are constructed with a resilient member or coil spring.

In further accordance with the disclosure, any prosthesis disclosed herein can be formed at least in part from a composite wire. In some embodiments, the composite wire can be drawn filled wire. For example, the drawn filled wire can include a first material, and a second material in a different region of the drawn filled wire that has greater radiopacity than the first material. The first and second materials can include metallic components and/or bioresorbable components. If desired, the second material can be located along a core region of the wire, and first material can surround or substantially surround the first material. The first material can include a NiTi alloy, and the second material can include platinum, for example. Other suitable examples for making such composite materials can be found in U.S. Patent Application Serial No. 10/524,387, filed September 13, 2004.

The devices disclosed herein can be implanted via the delivery system in transmural or transcameral applications using techniques similar to those presented in International Patent Application No. PCT/US2013/072344, filed November 27, 2013 and published February 12, 2015 as WO/2015/020682. A1. However, the presently disclosed embodiments permit easier deployment, adjustment, and retrievability by virtue of the elastic member and pushrod, among other things.

Thus, an exemplary method for use of any of the devices herein can be in conjunction with a method of transcatheter delivery of a device to the cardiovascular system. The method can include advancing a puncture device through a femoral vein to a venous crossing site, the venous crossing site being located along an iliac vein or the inferior vena cava. The method can further include using the puncture device to puncture a venous wall at the venous crossing site and then puncture an adjacent arterial wall at an arterial crossing site. The arterial crossing site is preferably located along an iliac artery or the abdominal aorta. The method can further include advancing at least a portion of the puncture device into the iliac artery or the abdominal aorta, thereby forming an access tract between the venous crossing site and the arterial crossing site.

The method can further include advancing a catheter through the access tract from the venous crossing site to the arterial crossing site, and delivering the device into the iliac artery or the abdominal aorta through the catheter. The device can be a prosthetic heart valve, aortic endograft, left ventricular assist device, or cardiopulmonary bypass device among other potential devices. In some embodiments, the puncture device can be selectively electrically energized to puncture the venous wall and the arterial wall. The puncture device can include inner and outer coaxial members, wherein the inner member comprises a guide wire or needle that is advanced to initially puncture the venous and arterial walls, and the outer member can be advanced over the inner member to enlarge the initial punctures and facilitate introduction of larger devices through the access tract. A target device can be advanced through a peripheral artery to adjacent the arterial crossing site. The target device can be used to guide an operator in directing the path of the puncture device through the arterial wall and into the iliac artery or the abdominal aorta.

After the access tract is formed, a guidewire can be introduced through the access tract. The catheter can then be advanced over the guidewire through the access tract into the iliac artery or the abdominal aorta to deliver the device. After delivering the device, an occlusion device as described herein can be delivered over a guidewire into the access tract to close the access tract. The occlusion device is preferably radially compressible for transcatheter delivery and radially expandable for implantation. The occlusion device can include an arterial portion for placement at the arterial crossing site, a venous portion for placement at the venous crossing site, and a neck portion for placement in the access tract. The occlusion device can include a guidewire channel extending through the venous portion, the neck portion, and the arterial portion. This portion of the procedure can be implemented by deploying a delivery catheter as disclosed herein and advancing it into the artery and deploying a first portion, such as a lobe or disc, of the prosthesis into the artery, optionally deploying one or more discs between the artery and vein, and deploying a disc or lobe into the vein. If the prosthesis includes a spring as described herein or tethers, the device can be collapsed by pushing on the push rod to partially collapse the prosthesis to permit it to be repositioned and redeployed, or fully collapsed and withdrawn back into the delivery system. The implant is preferably configured to be implanted across an arteriovenous fistula or tract connection between an artery and a vein with the arterial end portion positioned in the artery, wherein the venous end portion is positioned in the vein, and a neck portion is positioned in the fistula or tract connection.

The systems disclosed herein can be used to close congenital heart defects including atrial septal defect, ventricular septal defect, persistently patent ductus arteriosus. The system can be used to closed iatrogenic heart defects including extra-anatomic vascular access ports from the chest across the wall of the left or right ventricle into the respective lumen, or from the chest across the wall of the left or right atrium into the respective lumen, both to achieve temporary transcatheter access to the heart to allow therapeutic catheter interventional procedures or implantation such as mitral valve or tricuspid valve or aortic valve or pulmonic valve or prosthesis or annuloplasty implantation or modification or repair of Paravalvular leaks.

All statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

The methods and systems of the present disclosure, as described above and shown in the drawings, provide for improved techniques for treating lumenal systems of patients. It will be apparent to those skilled in the art that various modifications and variations can be made in the devices, methods and systems of the present disclosure Thus, it is intended that the present disclosure include modifications and variations that are within the scope of the appended claims.

## Claims

1. A prosthesis (100) for transcatheter correction of cardiovascular abnormalities comprising:
a radially expandable mesh body that is configured to self-expand into at least one disc (102, 112) after becoming radially unconstrained, the radially expandable mesh body defining a volume therein when expanded;
a tension coil spring (101) disposed within the volume of the mesh, the tension coil spring (101) being attached to a proximal end (114) and distal end (104) of the prosthesis (100) along an axis that defines a central region of the prosthesis (100), **characterized in** the tension coil spring (101) being configured to contract into a relaxed state when not being stretched that results in the prosthesis (100) being shortened along the axis and expanded radially.

2. The prosthesis of Claim 1, wherein the prosthesis (100) includes a woven graft material (108) and/or an elastomer with a coagulating coating disposed within the volume of the mesh that is configured to encourage coagulation when exposed to blood.

3. The prosthesis of Claim 2, wherein the graft material (108) disposed within the volume of the mesh includes first and second fabric discs (108a, 108c) that are disposed within the at least one disc (102) of the radially expandable mesh body to line the radially expandable mesh body with fabric.

4. The prosthesis of Claim 3, further comprising a tubular fabric portion (108e) attached to at least one of the fabric discs (108a, 108c), the tubular fabric portion (108e) extending proximally into a neck region (110) of the prosthesis (100).

5. The prosthesis of Claim 1, wherein the tension coil spring (101) includes at least two sections of different diameter.

6. The prosthesis of Claim 1, wherein the tension coil spring (101) includes at least three sections of different diameter.

7. The prosthesis of Claim 1, wherein the tension coil spring (101) includes at least three sections wherein each section has a diameter different than an adjacent section.

8. The prosthesis of Claim 1 wherein the tension coil spring (101) includes an enlarged central section for causing a neck region (110) of the prosthesis (100) to bulge radially outwardly when the prosthesis (100) is deployed.

9. The prosthesis of Claim 1, wherein the radially expandable mesh body is configured to self-expand into at least two discs (102, 112) connected by the neck region (110) after becoming radially unconstrained.

10. The prosthesis according to any of the preceding claims, wherein at least a portion of the prosthesis (100) is formed from a composite wire.

11. A system (200)for delivering a prosthesis, comprising:
a) an outer tubular sheath (124) having a proximal end and a distal end and defining a first lumen therethrough along its length;
b) an intermediate tubular member (118) disposed at least partially within the first lumen, and having a proximal end and a distal end and defining a second lumen therethrough along its length;
c) an inner elongate member (180)disposed at least partially within the second lumen, and having a proximal end and a distal end; and
d) a prosthesis(100)according to any of the preceding claims removably mounted on the distal end of the intermediate tubular member and wherein distal movement of the inner tubular member against a portion of the prosthesis causes it to collapse radially and elongate axially so as to permit the prosthesis to be collapsed after deployment, adjusted and redeployed or retracted into the outer tubular member and removed.

12. The system of Claim 11, wherein the inner elongate member (180) is a tubular member defining a guidewire lumen (106) therethrough.

13. The system of Claim 11, wherein a distal end (122) of the outer tubular member is cut at an angle that is oblique with respect to a central axis defined by the system.

## Patentansprüche

1. Prothese (100) zur Transkatheter-Korrektur von kardiovaskulären Anomalien, umfassend:
einen radial expandierbaren Netzkörper, der konfiguriert ist, um selbstständig zu mindestens einer Scheibe (102, 112) zu expandieren, nachdem er radial nicht mehr beschränkt ist, wobei der radial expandierbare Netzkörper im expandierten Zustand ein Volumen darin definiert;
eine Schraubenzugfeder (101), die innerhalb des Volumens des Netzes angeordnet ist, wobei die Schraubenzugfeder (101) an einem proximalen Ende (114) und einem distalen Ende (104) der Prothese (100) entlang einer Achse befestigt ist, die einen zentralen Bereich der Prothese (100) definiert, **dadurch gekennzeichnet, dass** die Schraubenzugfeder (101) konfiguriert ist, um sich in einen entspannten Zustand zusammenzuziehen, wenn sie nicht gedehnt wird, was dazu führt, dass die Prothese (100) entlang der Achse verkürzt und radial expandiert wird.

2. Prothese nach Anspruch 1, wobei die Prothese (100) ein gewebtes Transplantatmaterial (108) und/oder ein Elastomer mit einer koagulierenden Beschichtung einschließt, die innerhalb des Volumens des Netzes angeordnet ist, das konfiguriert ist, um die Koagulation zu fördern, wenn es Blut ausgesetzt wird.

3. Prothese nach Anspruch 2, wobei das innerhalb des Volumens des Netzes angeordnete Transplantatmaterial (108) erste und zweite Gewebescheiben (108a, 108c) einschließt, die innerhalb der mindestens einen Scheibe (102) des radial expandierbaren Netzkörpers angeordnet sind, um den radial expandierbaren Netzkörper mit Gewebe auszukleiden.

4. Prothese nach Anspruch 3, weiter umfassend einen röhrenförmigen Gewebeabschnitt (108e), der an mindestens einer der Gewebescheiben (108a, 108c) befestigt ist, wobei sich der röhrenförmige Gewebeabschnitt (108e) proximal in einen Halsbereich (110) der Prothese (100) erstreckt.

5. Prothese nach Anspruch 1, wobei die Schraubenzugfeder (101) mindestens zwei Teilabschnitte mit unterschiedlichem Durchmesser einschließt.

6. Prothese nach Anspruch 1, wobei die Schraubenzugfeder (101) mindestens drei Teilabschnitte mit unterschiedlichem Durchmesser einschließt.

7. Prothese nach Anspruch 1, wobei die Schraubenzugfeder (101) mindestens drei Teilabschnitte einschließt, wobei jeder Teilabschnitt einen anderen Durchmesser als ein benachbarter Teilabschnitt aufweist.

8. Prothese nach Anspruch 1, wobei die Schraubenzugfeder (101) einen vergrößerten Mittelabschnitt einschließt, um zu bewirken, dass sich ein Halsbereich (110) der Prothese (100) radial nach außen wölbt, wenn die Prothese (100) entfaltet wird.

9. Prothese nach Anspruch 1, wobei der radial expandierbare Netzkörper konfiguriert ist, um sich selbstständig zu mindestens zwei Scheiben (102, 112), die durch den Halsbereich (110) verbunden sind, zu expandieren, nachdem er radial nicht mehr beschränkt ist.

10. Prothese nach einem der vorstehenden Ansprüche, wobei mindestens ein Abschnitt der Prothese (100) aus einem Verbunddraht gebildet ist.

11. System (200) zum Einbringen einer Prothese, umfassend:
a) eine äußere röhrenförmige Ummantelung (124), die ein proximales und ein distales Ende aufweist und da hindurch ein erstes Lumen entlang ihrer Länge definiert;
b) ein röhrenförmiges Zwischenelement (118), das zumindest teilweise innerhalb des ersten Lumens angeordnet ist und ein proximales Ende und ein distales Ende aufweist und da hindurch ein zweites Lumen entlang seiner Länge definiert;
c) ein inneres längliches Element (180), das zumindest teilweise innerhalb des zweiten Lumens angeordnet ist und ein proximales Ende und ein distales Ende aufweist; und
d) eine Prothese (100) nach einem der vorstehenden Ansprüche, die entfernbar am distalen Ende des rohrförmigen Zwischenelements angebracht ist, und wobei eine distale Bewegung des inneren röhrenförmigen Elements gegen einen Abschnitt der Prothese bewirkt, dass sie radial kollabiert und axial verlängert wird, um zu gestatten, dass die Prothese nach dem Entfalten kollabiert, eingestellt und wieder entfaltet oder in das äußere rohrförmige Element zurückgezogen und entfernt werden kann.

12. System nach Anspruch 11, wobei das innere längliche Element (180) ein röhrenförmiges Element ist, das ein Führungsdrahtlumen (106) da hindurch definiert.

13. System nach Anspruch 11, wobei ein distales Ende (122) des äußeren röhrenförmigen Elements in einem Winkel geschnitten ist, der in Bezug auf eine durch das System definierte Mittelachse schräg verläuft.

## Revendications

1. Prothèse (100) pour la correction par transcathéter d'anomalies cardiovasculaires comprenant :
un corps de maille dilatable radialement qui est configuré pour s'auto-dilater en au moins un disque (102, 112) après être devenu radialement sans contrainte, le corps de maille dilatable radialement définissant un volume à l'intérieur de celui-ci lorsqu'il est dilaté ;
un ressort de traction hélicoïdal (101) disposé à l'intérieur du volume de la maille, le ressort de traction hélicoïdal (101) étant attaché à une extrémité proximale (114) et à une extrémité distale (104) de la prothèse (100) le long d'un axe qui définit une région centrale de la prothèse (100),
**caractérisée en ce que** le ressort de traction hélicoïdal (101) est configuré pour se contracter dans un état relâché lorsqu'il n'est pas étiré entraînant un raccourcissement de la prothèse (100) le long de l'axe et sa dilatation radiale.

2. Prothèse selon la revendication 1, dans laquelle la prothèse (100) inclut un matériau de greffe tissé (108) et/ou un élastomère doté d'un revêtement de coagulation disposé à l'intérieur du volume de la maille qui est configuré pour favoriser la coagulation lorsqu'il est exposé à du sang.

3. Prothèse selon la revendication 2, dans laquelle le matériau de greffe (108) disposé à l'intérieur du volume de la maille inclut des premier et second disques de tissu (108a, 108c) qui sont disposés à l'intérieur du au moins un disque (102) du corps de maille dilatable radialement pour tapisser le corps de maille dilatable radialement avec du tissu.

4. Prothèse selon la revendication 3, comprenant en outre une partie tissulaire tubulaire (108e) attachée à au moins l'un des disques de tissu (108a, 108c), la partie tissulaire tubulaire (108e) s'étendant proximalement dans une région col (110) de la prothèse (100).

5. Prothèse selon la revendication 1, dans laquelle le ressort de traction hélicoïdal (101) inclut au moins deux sections de diamètre différent.

6. Prothèse selon la revendication 1, dans laquelle le ressort de traction hélicoïdal (101) inclut au moins trois sections de diamètre différent.

7. Prothèse selon la revendication 1, dans laquelle le ressort de tension hélicoïdal (101) inclut au moins trois sections, dans laquelle chaque section a un diamètre différent d'une section adjacente.

8. Prothèse selon la revendication 1 dans laquelle le ressort de traction hélicoïdal (101) inclut une section centrale élargie pour amener une région col (110) de la prothèse (100) à faire saillie radialement vers l'extérieur lorsque la prothèse (100) est déployée.

9. Prothèse selon la revendication 1, dans laquelle le corps de maille dilatable radialement est configuré pour s'auto-dilater en au moins deux disques (102, 112) connectés par la région col (110) après être devenu radialement sans contrainte.

10. Prothèse selon l'une quelconque des revendications précédentes, dans laquelle au moins une partie de la prothèse (100) est formée d'un fil composite.

11. Système (200) pour poser une prothèse, comprenant :
a) une gaine tubulaire externe (124) ayant une extrémité proximale et une extrémité distale et définissant une première lumière à l'intérieur de celle-ci le long de sa longueur ;
b) un membre tubulaire intermédiaire (118) disposé au moins en partie à l'intérieur de la première lumière et ayant une extrémité proximale et une extrémité distale et définissant une seconde lumière à l'intérieur de celui-ci le long de sa longueur ;
c) un membre allongé interne (180) disposé au moins en partie à l'intérieur de la seconde lumière et ayant une extrémité proximale et une extrémité distale ; et
d) une prothèse (100) selon l'une quelconque des revendications précédentes montée de manière amovible sur l'extrémité distale du membre tubulaire intermédiaire et dans lequel le mouvement distal du membre tubulaire interne contre une partie de la prothèse provoque son affaissement radial et son allongement axial de façon à permettre à la prothèse de s'affaisser après le déploiement, d'être ajustée et redéployée ou rétractée dans le membre tubulaire externe, et retirée.

12. Système selon la revendication 11, dans lequel le membre allongé interne (180) est un membre tubulaire définissant une lumière de fil-guide (106) à l'intérieur de celui-ci.

13. Système selon la revendication 11, dans lequel une extrémité distale (122) du membre tubulaire externe est coupée selon un angle qui est oblique par rapport à un axe central défini par le système.
